# EUROPEAN PATENT SPECIFICATION

(11) **EP 1 718 258 B1**
(45) Date of publication and mention of the grant of the patent: **25.03.2009**
(21) Application number: 05713575.8
(22) Date of filing: 15.02.2005
(51) Int. Cl.: A61K 9/16, A61K 9/70, A61K 31/485

(54) **ABUSE RESISTANCE OPIOID TRANSDERMAL DELIVERY DEVICE**
MISSBRAUCHSICHERE TRANSDERMALE ABGABEVORRICHTUNG FÜR OPIOIDE, ENTHALTEND OPIOIDANTAGONIST IN FORM VON MIKROKÜGELCHEN
DISPOSITIF DE DISTRIBUTION TRANSDERMIQUE D'OPIOIDES EMPECHANT UNE UTILISATION ABUSIVE ET CONTENANT DES MICROSPHERES D'ANTAGONISTES D'OPIOIDES

(30) Priority: 23.02.2004 US 547196 P
(43) Date of publication of application: 08.11.2006
(73) Proprietor: EURO-CELTIQUE S.A., 1653 Luxembourg (LU)
(72) Inventor: REIDENBERG, Bruce, Rye, New York NY 10580 (US); SHEVCHUK, Ihor, Yonkers, NY 10710 (US); TAVARES, Lino, Kinnelon, NJ 07405 (US); LONG, Kevin, Oak Ridge, NJ 07438 (US); MASKIEWICZ, Richard, Redlands, CA 92373 (US); SHAMEEM, Mohammed, Nanuet, NY 10954 (US)
(74) Representative: Maiwald, Walter
(86) International application number: PCT/US2005/004741
(87) International publication number: WO 2005/081825

(56) References cited:
- EP-A1- 0 330 180
- WO-A-00/61147
- WO-A-01/58447
- WO-A-99/32119
- WO-A-02/087482
- WO-A-02/094172
- WO-A-20/04014336
- US-A- 5 149 538
- US-A- 5 236 714
- US-A1- 2002 010 127
- US-A1- 2002 187 183
- US-A1- 2003 124 061
- US-A1- 2004 033 255

## Description

### FIELD OF THE INVENTION

The present invention relates to transdermal delivery devices useful for delivering an opioid agonist while decreasing the potential for abuse.

### BACKGROUND OF THE INVENTION

Sustained-release formulations of opioids are known in the art and provide a longer period of pharmacologic effect than is ordinarily experienced after the administration of immediate release preparations of the opioid. Such longer periods of efficacy achieved with sustained-release formulations can provide many therapeutic benefits that are not achieved with corresponding immediate release preparations.

One approach to sustained delivery of a therapeutically active agent is the use of a transdermal delivery device, such as a transdermal patch. Certain commercially available transdermal devices that deliver, e.g., scopolamine or nitroglycerin, comprise a reservoir sandwiched between an impervious backing and a membrane face, and are usually attached to the skin by an adhesive gel.

In recent years, transdermal administration has gained increasing acceptance in the management of chronic pain syndromes; for example, when around-the-clock analgesia is indicated. Transdermal delivery devices in which an opioid analgesic is the active ingredient are known. Generally, a transdermal delivery device contains a therapeutically active agent (e.g., an opioid analgesic) in a reservoir or matrix, and an adhesive which enables the transdermal device to adhere to the skin, allowing for the passage of the active agent from the device through the skin of the patient. Once the active agent has penetrated the skin layer, the drug is absorbed into the blood stream where it can exert a desired pharmacotherapeutic effect such as analgesia. Examples of patents in this area include U.S. Patent No. 4,588,580 to Gale, which describes transdermal delivery devices for the delivery of fentanyl or its analgesically effective derivatives; U.S. Patent No. 5,908,846 to Bundgaard, which describes a topical preparation of derivatives of morphine in association with a carrier in the form of a transdermal patch; U.S. Patent No. 4,806,341 to Chien et al., which describes transdermal administration of narcotic analgesics or opioid antagonists using a device comprising a backing layer, an adjoining layer of a solid polymer matrix containing morphinan narcotic analgesics or antagonists and skin permeation enhancers, and an adhesive polymer; U.S. Patent No. 4,626,539 to Aunst et al., which describes transdermal patches containing a gel, lotion or cream composed of an opioid, a penetration enhancer, and a pharmaceutical carrier such as propylene glycol; and U.S. Patents Nos. 5,968,547, 6,231,886, and 6,344,212 to Reder et al., which describe transdermal delivery devices containing buprenorphine to provide prolonged pain management.

A commercially available opioid analgesic transdermal device marketed in the United States is the Duragesic^{®} patch, which contains fentanyl as the active agent (commercially available from Janssen Pharmaceutical). The Duragesic^{®} patch is adapted to provide analgesia for up to 48 to 72 hours.

A major concern associated with the use of opioids is the abuse of such drugs, and the diversion of these drugs from a patient in need of such treatment to a non-patient, e.g., to a non-patient for illicit use. It has been recognized in the art that transdermal opioid formulations may be abused when the delivery device is tampered with (e.g., by chewing, tearing, or extracting the drug) in order to liberate the opioid for illicit use (e.g., for oral or parenteral use). In addition, there have been reports of previously "used" transdermal fentanyl delivery devices being subsequently abused for their overage.

U.S. Patent No. 5,236,714 to Lee et al. and U.S. Patent No. 5,149,538 to Granger et al. describe opioid agonist transdermal delivery devices purportedly having decreased potential for abuse.

There exists a need for a transdermal opioid delivery device having a decreased potential for abuse of the opioid contained in the device.

### OBJECTS AND SUMMARY OF THE INVENTION

It is an object of the present invention to provide a transdermal delivery device containing an opioid analgesic, and having reduced potential for abuse.

It is a further object of the present invention to provide a method of treating pain with an opioid-containing transdermal delivery device having reduced potential for abuse.

In accordance with the above objects and others, the present invention is directed to a transdermal delivery device as defined in claim 1.

In certain embodiments, the present invention is directed to a transdermal delivery device comprising a drug containing layer comprising an effective amount of an opioid agonist and a plurality of microspheres dispersed in the drug containing layer, the microspheres comprising an opioid antagonist and being visually indiscernible in the drug containing layer

In certain embodiments, the present invention is directed to a transdermal delivery device comprising a backing layer; and a drug-containing layer in contact with one surface of the backing layer, the drug-containing layer comprising an effective amount of an opioid agonist and a plurality of microspheres dispersed in the drug-containing layer, the microspheres comprising an opioid antagonist and a polymer selected from the group consisting of polyesters, polyethers, poly(orthoesters), polysaccharides, cyclodextrins, chitosan, poly (Σ-caprolactones), polyanhydrides, albumin, blends and copolymers thereof, the microspheres in a mean size of from 1 to 300 µm.

In certain embodiments, the present invention is further directed to a transdermal delivery device comprising a backing layer; and a drug-containing layer in contact with one surface of the backing layer, the drug-containing layer comprising an effective amount of an opioid agonist and a plurality of microspheres dispersed in the drug-containing layer, the microspheres comprising an opioid antagonist dispersed in a polymeric matrix, the microspheres in a mean size of from 1 to 300 µm.

In certain embodiments, the present invention is directed to a transdermal delivery device comprising a backing layer; and a drug containing layer connected to one surface of the backing layer, the drug containing layer comprising an effective amount of an opioid agonist and a plurality of microspheres dispersed in the drug containing layer, the microspheres in a mean size of from 1 to 300 µm and comprising an opioid antagonist. In such an embodiment, the size of the antagonist containing microspheres will not easily be separated from the opioid agonist by an abuser in an attempt to abuse the opioid agonist contained in the transdermal device.

In certain embodiments, the present invention is directed to a transdermal delivery device comprising a backing layer; and a drug-containing layer in contact with one surface of the backing layer, the drug-containing layer comprising an effective amount of an opioid agonist and a plurality of microspheres dispersed in the drug-containing layer, the microspheres consisting essentially of an opioid antagonist and a polymer selected from the group consisting of polyesters, polyethers, poly(orthoesters), polysaccharides, cyclodextrins, chitosans, poly (Σ-caprolactones), polyanhydrides, albumin, blends and copolymers thereof.

In certain embodiments, the present invention is further directed to a transdermal delivery device comprising a backing layer; and a drug-containing layer in contact with one surface of the backing layer, the drug-containing layer comprising an effective amount of an opioid agonist and a plurality of microspheres dispersed in the drug-containing layer, the microspheres consisting essentially of an opioid antagonist dispersed in a polymeric matrix.

In certain embodiments, the opioid agonist-containing layer is selected from an adhesive layer, a matrix layer, a reservoir, or a combination thereof.

In certain embodiments, the antagonist is non-releasable or substantially non-releasable from the microspheres (and therefore not released or not substantially released from the device) when the transdermal delivery device is applied topically and intact to the skin of a human patient. The antagonist, however, is releasable from the microspheres when the transdermal delivery device is tampered with, e.g., chewed, soaked, punctured, torn, or otherwise subjected to any other treatment that disrupts the integrity of the microspheres.

In certain preferred embodiments, the microspheres of the present invention which are dispersed in the matrix layer containing the opioid agonist have a similar visual appearance to other components of the matrix layer (e.g., the opioid agonist, the polymer(s), etc.) so that the opioid agonist and opioid antagonist cannot be readily identified by visual inspection, thereby increasing the difficulty in separation of the opioid agonist from the antagonist.

In certain preferred embodiments, the composition of the matrix layer inhibits the dissolution of the microspheres and the release of the opioid antagonist upon the intact topical application of the device to the skin of a human patient.

In the present invention, the amount of antagonist released from a transdermal ' delivery device of the present invention that has been tampered with (e.g., chewed, soaked, punctured, torn, or subjected to any other treatment disrupting the integrity of the microspheres) is an amount that at least partially blocks the opioid agonist when administered (e.g., orally, intranasally, parenterally or sublingually). Preferably, the euphoric effect of the opioid agonist will be attenuated or blocked, thereby reducing the tendency for misuse and abuse of the dosage form.

Physico/chemical features of the polymers can be utilized to provide abuse resistance of the present invention. For example, hydrolysis of poly (orthoester) is catalyzed by acid. Thus, abuse via oral ingestion of the opioid-containing portion of the transdermal delivery device containing microspheres comprising poly(orthoester) and opioid antagonist would result in degradation of the polymer and the release of the opioid antagonist in the acid milieu of the stomach. In addition, degradation of microspheres comprising polysaccharides and proteins is catalyzed by enzymatic cleavage. Thus, abuse via oral ingestion of a transdermal delivery device of microspheres comprising dextrans would result in degradation of the polymer and release of the opioid antagonist in the gastrointestinal tract. Further, an abuser might try to extract a transdermal formulation containing microspheres by immersing the entire formulation in diethyl ether. The microspheres would dissolve in the ether releasing the antagonist, rendering the liquid unsuitable for abuse. In a further embodiment, in the setting of intraoral abuse of a transdermal dosage form, saliva would penetrate the transdermal formulation and dissolve the microspheres, releasing the antagonist and decreasing the value of the transdermal formulation to the abuser. In such an embodiment, the micropsheres could comprise a material such as starch which is degraded by salivary amylase.

In certain preferred embodiments, a separate adhesive layer may be included in contact with the matrix layer opposite that side of the matrix layer in contact with the _ backing layer. In other preferred embodiments, the matrix layer containing the opioid agonist and microspheres of antagonist comprises a pharmaceutically acceptable polymer that also acts as a transdermal adhesive, and no additional adhesive layer is necessary to enable the transdermal device to adhere to a patient's skin. In certain preferred embodiment, the adhesive layer used to affix the transdermal delivery device to the skin of the patient comprises a pressure sensitive adhesive. In certain embodiments, the transdermal delivery device further comprises a removable protective layer that is in contact with the matrix or adhesive layer and that is removed prior to application of the transdermal delivery device to the skin.

In preferred embodiments, the transdermal delivery device provides effective pain management for a period of 2 to 8 days when worn intact on the skin of a human patient.

In certain embodiments, the transdermal delivery device is a transdermal patch, a transdermal plaster, a transdermal disc, an iontophoretic transdermal device, or the like.

The term "sustained release" is defined for purposes of the present invention as the release of the opioid agonist from the transdermal delivery device at such a rate that blood (e.g., plasma) concentrations (levels) are achieved and maintained within the therapeutic range but below toxic levels over at least 1 day and, e.g., for 2 to 8 days.

For purposes of the present invention, the term "opioid agonist" is interchangeable with the term "opioid" or "opioid analgesic" and includes the base of the opioid and pharmaceutically acceptable salts thereof. The present invention also contemplates the administration of a prodrug thereof (e.g., ethers or esters) that is converted to an active agonist in the patient's device. The opioid agonist may be a full agonist, a mixed agonist-antagonist, or a partial agonist.

For purposes of the present invention, the term "opioid antagonist" includes the base of the antagonist and pharmaceutically acceptable salts thereof. The present invention also contemplates the administration of a prodrug thereof. Examples of opioid antagonists include, e.g., nalorphine, nalorphine dinicotinate, naloxone, nalmephene, cyclazocine, levallorphan, naltrexone, nadide, cyclazocine, amiphenazole and pharmaceutically acceptable salts thereof and mixtures thereof

The term "effective analgesia" is defined for purposes of the present invention as a satisfactory reduction in, or elimination of, pain as determined by a human patient or through use of a recognized pain scale. In a preferred embodiment, effective analgesia is not accompanied by any side effects, or is accompanied by a tolerable level of side affects, as determined by a human patient.

The term "microsphere" as used herein means solid (or semi-solid) particles containing an active agent dispersed in (matrix type), or coated by (microcapsule), a biocompatible polymer that serves to render the antagonist non-releasable or substantially non-releasable. The term "substantially non-releasable" means that the antagonist might be released in a small amount, as long as the amount released does not affect or does not significantly affect analgesic efficacy when the dosage form is administered transdermally as intended.

The term "flux" refers to the rate of penetration of a chemical entity, such as an opioid agonist or opioid antagonist, through the skin of an individual.

The term "emulsion" for the purposes of the present invention means a stable dispersion of one liquid in a second immiscible liquid. With respect to emulsions, the term "continuous phase" means the external phase, as compared to the "dispersed phase" which is the internal phase. For example, if an emulsion is a "water-in-oil" (w/o) emulsion, the oil is the continuous phase, whereas in an "oil-in-water" (o/w) emulsion, water is the continuous phase.

The term "pharmaceutically acceptable salt" means any non-toxic, suitable salt of an opioid agonist or antagonist having therapeutic properties in a mammal, particularly a human. Preparation of such salts is known to those skilled in the pharmaceutical arts. Useful salt forms of opioid agonists or opioid antagonists may include, for example, the hydrochloride, hydrobromide, hydroiodide, sulfate, bisulfate, nitrate, citrate, tartrate, bitartrate, lactate, phosphate, maleate, fumarate, succinate, acetate, palmeate, stearate, oleate, palmitate, napsylate, tosylate, methane sulfonate, succinate, laurate, valerate salts among others.

### BRIEF DESCRIPTION OF THE FIGURES

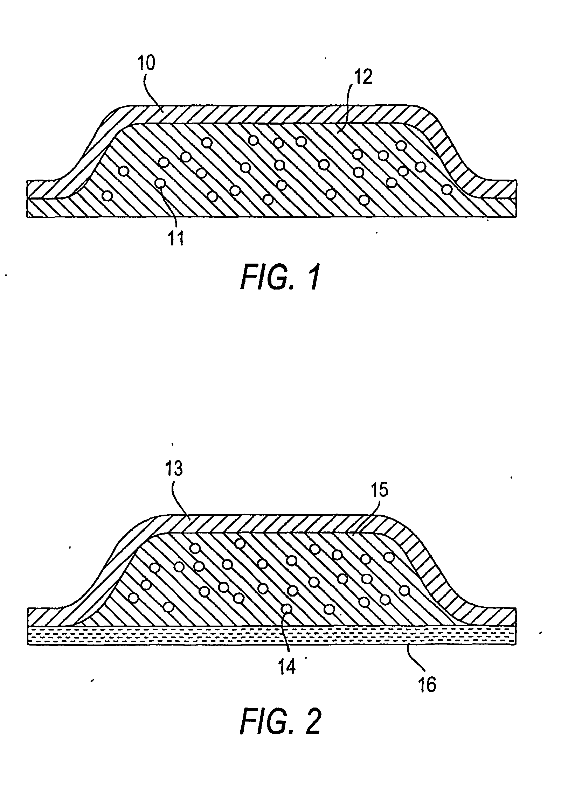

Fig. 1 shows a cross-section of one embodiment of a transdermal delivery device of the present invention. The device has an impermeable backing layer **10,** such as a metal foil, plastic film, or a laminate of different materials. In contact with and beneath backing layer **10** is located a matrix layer containing both opioid agonist and microspheres **11** containing polymer and opioid antagonist. The matrix layer of this embodiment acts as both a reservoir for the opioid agonist and an adhesive, enabling this transdermal delivery device to adhere to the skin of a human patient.

Fig. 2 shows a cross-section of one embodiment of a transdermal delivery device of the present invention. The device is similar to the device shown in Fig 1 since it has an impermeable backing layer **13** and a matrix layer **15** in contact with and beneath the backing layer **13.** The matrix layer contains both opioid agonist and microspheres **14** containing polymer and opioid antagonist. This transdermal delivery device also has a separate adhesive layer **16** in contact with the matrix layer and in contact with certain parts of the backing layer, enabling this transdermal delivery device to adhere to the skin of a human patient.

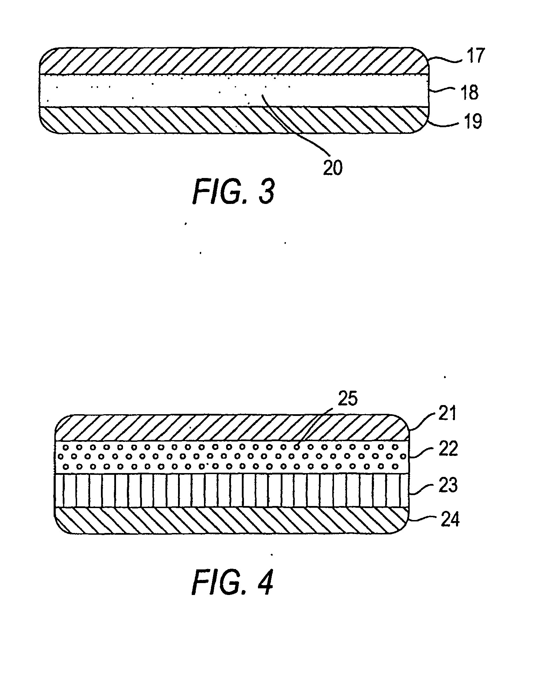

Fig. 3 shows a cross-section of one embodiment of a transdermal delivery device of the present invention. The device has an impermeable backing layer **17** and a matrix layer **18** in contact with and beneath backing layer 17. The matrix layer contains opioid agonist and microspheres **20** containing polymer and opioid antagonist. The matrix layer acts as an adhesive, enabling the transdermal delivery device to adhere to the skin of a human patient. This transdermal delivery device also has a removable protective layer **19** in contact with and beneath the matrix layer which is removed prior to application of the transdermal delivery device.

Fig. 4 shows a cross-section of one embodiment of a transdermal delivery device of the present invention. The device is similar to the device shown in Fig. 3, in that it has an impermeable backing layer **21** and a matrix layer **22** in contact with and beneath backing layer **21.** The matrix layer contains opioid agonist and microspheres **25** containing polymer and opioid antagonist. In addition, this transdermal delivery device has an adhesive layer **23** in contact with and beneath the matrix layer **22,** enabling the transdermal delivery device, to adhere to the skin of a human patient. This transdermal delivery device also has a removable protective layer **24** in contact with and beneath the adhesive layer, which is removed prior to application of the transdermal delivery device.

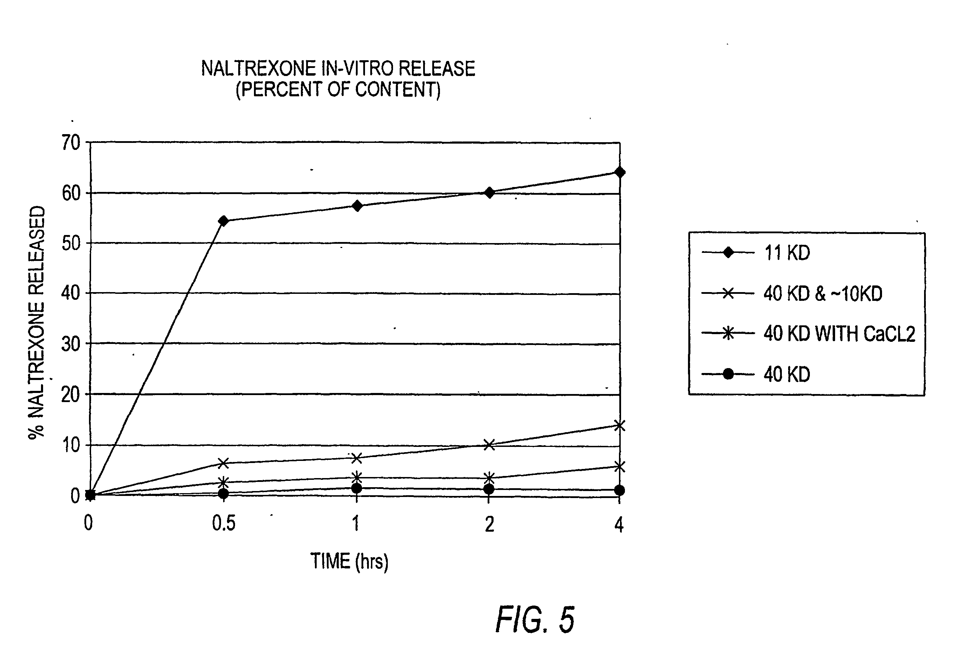

Figure 5 depicts in-vitro release of naltrexone from microspheres prepared in accordance with Example 1.

### DETAILED DESCRIPTION

Certain devices prepared and used according to the present invention contain an opioid antagonist dispersed in microspheres. In certain embodiments, the amount of the opioid antagonist incorporated into the microspheres ranges from about 1% by weight to about 90% by weight, or from about 5% by weight to about 70% by weight, or from about 30% to about 50% by weight of the microsphere (including active).

In the present invention, the opioid antagonist is incorporated into microspheres for use in opioid transdermal delivery devices in order to make the opioid antagonist non-releasable or substantially non-releasable upon topical application of an intact transdermal delivery device comprising the antagonist microspheres. The microspheres preferably comprise a polymeric substance. Suitable polymers that can be used to form opioid-containing antagonist microspheres include soluble, insoluble, biodegradable, and non-biodegradable polymers. The use of pharmaceutically acceptable non-toxic polymers is preferred.

Physicochemical features of the polymers can be selected to provide further abuse resistance of the present invention. For example, hydrolysis of poly (orthoester) is catalyzed by acid. Thus, abuse via oral ingestion of the opioid-containing portion of a transdermal delivery device containing microspheres of poly(orthoester) comprising opioid antagonist would result in degradation of the polymer and release of the opioid antagonist in the acid milieu of the stomach. Degradation of microspheres comprising polysaccharides and proteins is catalyzed by enzymatic cleavage. Thus, for example, abuse via oral ingestion of the opioid-containing portion of the transdermal delivery device containing microspheres of dextrans would result in degradation of the polymer and release of the opioid antagonist in the gastrointestinal tract.

Polymers that may be used for the opioid antagonist-containing microspheres of the present invention can generally be classified into three types, namely natural, semi-synthetic and synthetic, based on their sources. The natural biodegradable polymers may be further classified into proteins and polysaccharides.

Representative natural derived polymers include proteins, such as zein, modified zein, casein, gelatin, gluten, albumin, fetuin, orosomucoid, glycoproteins, collagen, synthetic polypeptides and elastin. Biodegradable synthetic polypeptides include, for example, poly-(N-hydroxyalkyl)-L-asparagine, poly-(N-hydroxyalkyl)-L-glutamine, and copolymers of N-hydroxyalkyl-L-asparagine and N-hydroxyalkyl-L-glutamine with other amino acids, e.g., L-alanine, L-lysine, L-phenylalanine, L-valine, L-tyrosine, and the like. Polysaccharides (e.g., cellulose, dextrans, polyhyaluronic acid, lipopolysaccharides), polymers of acrylic and methacrylic esters, and alginic acid, may also be used.

Synthetically modified, natural (i.e., semi-synthetic) polymers include alkyl celluloses, hydroxyalkyl celluloses, cellulose ethers, cellulose esters, and nitrocelluloses, among others.

Semi-synthetic biodegradable, polymers are produced by modifying natural polymers to produce polymers having altered physicochemical properties such as thermogelling properties, mechanical strength and degradation rates. Examples of semi-synthetic, biodegradable polymers suitable for use in the present invention include modified chitosan complexes, chondroitin sulfate-A chitosan complexes, and water soluble, phosphorylated chitosans (P-chitosans), and combinations thereof, such as, for example, alginate-chitosan.

Lack of immunogenicity and more reproducible and predictable physicochemical properties make synthetic, biodegradable polymers preferable to the natural polymers for drug delivery uses. These polymers may be non-toxic and biodegradable, and delivery devices have been prepared from these polymers. Therefore, synthetic biodegradable polymers may be particularly suitable for the microspheres of the present invention

Non-limiting examples of synthetic biodegradable polymers include: polyesters, polyethers, poly(orthoesters), poly(vinyl alcohols), polyamides, polycarbonates, polyacrylamides, polyalkylene glycols, polyalkylene oxides, polyalkylene terephthalates, polyvinyl ethers, polyvinyl esters, polyvinyl halides, polyvinylpyrrolidone, polyglycolides, polysiloxanes, polylactides, polyurethanes and copolymers thereof Non-limiting examples of polyesters include polylactic acid, polyglycolic acid, poly(lactide-co-glycolide), poly(e-caprolactone), polydioxanone, poly(ethylene terephthalate), poly(malic acid), poly(tartronic acid), polyphosphazenes, poly(orthoester), poly(valeric acid), poly(buteric acid), polyliydroxybutyrate, polyhydroxyvalerate, polyanhydride, and copolymers of the monomers used to synthesize any of the above-mentioned polymers, e.g., poly(lactic-co-glycolic acid) or the copolymer of polyhydroxy butyrate with hydroxyvaleric acid (Biopol^{®} by Zeneca). Copolymers of lactic and glycolic acids, e.g., poly(lactic-co-glycolic acid) (PLGA), have been extensively studied for their use in drug delivery devices such as microspheres.

In certain embodiments, the polymer (e.g., PLGA) can have a molecular weight from about 1 KD to about 100 KD or greater, from about 5 KD to about 60 KD or from about 10 KD to about 40 KD. In certain embodiments, a portion of the PLGA (e.g., from 10% to about 90%) can have a molecular weight of less than 20 KD, or less than 15 KD and the corresponding, remaining portion (e.g., from 90% to 10%) can have a molecular weight of greater than 25 KD, or greater than 35 KD.

Poly(e-caprolactone) may be used in preparing microspheres for use in the present invention. The degradation rate of poly(e-caprolactone) is much slower than that of either polyglycolic acid or poly(lactic-co-glycolic acid). Poly(e-caprolactone) has exceptional ability to form blends with many other polymers. Copolymers of poly (e-caprolactone) can be used to control permeability and mechanical properties of drug delivery devices.

Polyethers and poly(orthoesters) may also be used in preparing microspheres for use in the present invention. These polymers have been incorporated into multiblocks for block polymers having diverse degradation rates, mechanical strength, porosity, diffusivity, and inherent viscosity. Examples of polyethers include polyethylene glycol and polypropylene glycol. An example of a multiblock copolymer is poly(ether ester amide). Additionally, triblock copolymers of poly(orthoesters) with various poly(ethylene glycol) contents are useful for their stability in water/oil (w/o) emulsions, and possess greater efficacy than poly(orthoester) when used in preparing microspheres. Other useful block copolymers include diblock copolymers of poly (lactic-co-glycolic acid) and poly(ethylene glycol) (PEG), triblock copolymers of PEG-PLGA-PEG, copolymers of PLGA and polylysine, and poly (ester ether) block copolymers.

Microspheres useful in practicing the present invention are spherically shaped and from 1 to 300 microns, from 1 to 200 microns, from 1 to 100 microns, from 125 to 200 microns, or from 50 to 100 microns in diameter. Microsphere size may be dependent upon the type of polymer used. In certain embodiments, rather than being spherical, the microspheres may be irregularly shaped, wherein the diameter is considered to be the largest cross-section of the microsphere.

In certain embodiments, the microspheres used in the present invention comprise opioid antagonist in an amount of from about 5% to about 70% by weight of the microsphere (including active).

In certain embodiments, the opioid antagonist can be loaded into the microspheres via microencapsulation. Techniques for microencapsulation for use in accordance with the present invention are described in U.S. Patent Nos. 3,161,602; 3,396,117; 3,270,100; 3,405,070; 3,341,466; 3,567,650; 3,875,074; 4,652,441; 5,100,669; 4,438,253; 4,391,909; 4,145,184; 4,277,364; 5,288,502; and 5,665,428. Furthermore, the microspheres can be prepared by either solvent evaporation as described, e.g., by E. Mathiowitz, et al., J. Scanning Microscopy, 4, 329 (1990); L.R. Beck, et al., Fertil. Steril., 31, 545 (1979); and S. Benita, et al, J. Pharm.Sci. 73, 1721 (1984); or by hot-melt microencapsulation, as described, e.g., by E. Mathowitz, et al., Reactive Polymers 6, 275 (1987); or by spray drying. The microspheres may be prepared by any method known in the art including but not limited to coacervation, phase-separation, solvent evaporation, spray-drying, spray-congealing, pan-coating, fluid bed coating or the like.

For purposes of the present invention, a microcapsule can be described functionally as a small container enclosing the contents with a film. The film may be made of synthetic, semi-synthetic, or natural polymer as described above, and can control the release (or provide no release) of the contents. The release rate of the contents from a microcapsule is mainly determined by the chemical structure and thickness of the capsule film and size of the microcapsule. In microcapsule formulations, small solid particles of opioid antagonist can be coated with a coating which consists of an organic polymer, hydrocolloid, sugar, wax, fat, metal, or inorganic oxide.

In certain embodiments, the opioid antagonist is incorporated into the microspheres using an oil/water (o/w) emulsion, a water/oil (w/o) emulsion, an oil/oil (o/o) emulsion, an oil/water/oil (o/w/o) emulsion, an oil/water/water (o/w/w) emulsion, water/oil/water (w/o/w) emulsion, or a water/oil/oil (w/olo) emulsion, or the like.

In certain embodiments, the opioid antagonist is incorporated into the microspheres by microemulsification of a fixed oil and an aqueous solution of a water-soluble opioid antagonist. This emulsion is of the "water in oil" type. When the emulsion is of the "water-in-oil" type, oil is the continuous phase or external phase and water is the "dispersed" or internal phase as opposed to the "oil in water" device, where water is the continuous phase.

In certain preferred embodiments, the opioid antagonist may be incorporated into the microspheres via a multi-phase emulsification device such as w/o/w. The opioid antagonist may be incorporated into multi-phase microspheres prepared by a multiple emulsion solvent evaporation technique. In this technique, the opioid antagonist is incorporated into biodegradable polymeric microsphere by an emulsification process. The device is suitable for both water soluble and insoluble opioid antagonists.

The microspheres of the present invention may be multiphasic polymeric microspheres in which the opioid antagonist is dispersed in oily droplets in a polymeric matrix. The microspheres can be prepared by a multiple emulsion solvent evaporation technique as described in U.S. Patent No. 5,288,502. This patent describes a multiple emulsion solvent technique, where the drug is protected within an oily droplet and contact with the polymer, organic solvent, and other potentially denaturing agents is avoided.

Multiple emulsions are devices in which drops of the oil-dispersed phase themselves contain even smaller aqueous dispersed droplets consisting of a liquid identical with the aqueous continuous phase. They are emulsions of emulsions with high capacity for entrapment of drug, protection of the entrapped drug, ability to introduce incompatible substances into the same device, and prolongation of release.

Any of a variety of fixed oils may be used in preparing the microspheres, including safflower, soybean, peanut, cotton seed, sesame, or cod liver oil, among others. In certain preferred embodiments, soybean, sesame or safflower oil are used. The oil phase may consist of isohexadecane or liquid paraffin. Oil concentration influences the stability of the emulsion. Stability is optimal with an oil percentage preferably in a range of 20-30% w/w of the total emulsion.

In the multiple emulsion process, the organic phase may be prepared by preparing an emulsion containing the opioid antagonist and a polymeric material. Preferably, the opioid antagonist is dispersed in an organic polymer solution in either methylene chloride or ethyl acetate. The resulting primary w/o emulsion is then dispersed into an external aqueous phase to form a second emulsion that comprises microspheres containing the opioid antagonist in the polymeric matrix material (i.e., emulsification into the external phase). The subsequent process steps are similar to the o/w method. The step of dissolving the drug into the internal aqueous phase is eliminated. In addition, higher theoretical drug loading is achieved because the internal drug phase consists only of solid particles and not of a drug solution.

In yet other embodiments, an o/w emulsion process may be used to incorporate the opioid antagonist into the microspheres. For the o/w dispersion method, the opioid antagonist is dispersed in the polymer phase followed by emulsification in the external aqueous phase. The microspheres are then separated from the external aqueous phase by wet sieving, followed by washing and desiccator-drying.

In certain embodiments, the present invention utilizes encapsulation techniques that allow liquid or solid substances to be encapsulated by polymers. In certain preferred embodiments, the opioid antagonist is in crystalline form. The crystalline opioid antagonist particles may be formed by solid-state crystallization via exposure to solvent vapors. The crystalline form may decrease the water content of the preparation, thus preserving the stability of the opioid antagonist. The crystal may be encapsulated in a fixed oil, and mixed with a solution of polymer and solvent in dispersion oil. U.S. Patent No. 6,287,693 to Savoir describes stable shaped particles of crystalline organic compounds that are formed into microspheres and achieve storage stability. Alternatively, any suitable method for producing crystalline particles of organic compounds can be used.

The stability and release characteristics of emulsion devices are influenced by a number of factors such as the composition of the emulsion, droplet size, viscosity, phase volumes and pH. The encapsulation efficacy of the opioid antagonist can be optimized by minimizing the migration of drug from the polymer by altering the volume, temperature and chemical composition of the extraction medium (quench solution) during the encapsulation process. The purpose of the quench solution is to remove most of the organic solvent from the microspheres during processing.

The quench liquid can be plain water, an aqueous solution, or other suitable liquid, the volume, amount, and type of which depends on the solvents in the emulsion phase. The quench liquid volume is on the order of 10 times the saturated volume (i.e., 10 times the quench volume needed to completely absorb the volume of solvent in the emulsion). The quench volume can vary from about 2 to about 20 times the saturated volume.

After quenching, the microspheres are separated from the aqueous quench solution by, e.g., decantation or filtration with a sieve column. Various other separation techniques can be used.

Residual solvent in the microspheres accelerates the degradation process, thereby reducing their shelf-life. The microspheres are therefore preferably washed with water or a solvent miscible therewith to further remove residual solvent, preferably to a level of about 0.2 to about 2.0% or less. Aliphatic alcohols such as methanol, ethanol, propanol, butanol, and isomers of the foregoing are preferred for use in the wash solution. Most preferred is ethanol.

Alternatively, solvent removal can be accomplished by evaporation. In embodiments where the solvent evaporation method is used, the polymer can be dissolved in a volatile organic solvent. The opioid antagonist is dispersed or dissolved in a solution of the selected polymer and a volatile organic solvent like methylene chloride, the resultant dispersion or solution is suspended in an aqueous solution that contains a surface active agent such as poly (vinyl alcohol), and a temperature gradient is used to remove the solvent.

The solvent evaporation method may involve dissolving the opioid antagonist and polymer in a co-solvent device. However, alternative methods may be used that omit the incorporation of unacceptable organic solvents. The resulting emulsion is stirred until most of the organic solvent is evaporated, leaving solid microspheres. The solution can be loaded with the opioid antagonist and suspended in 200 ml of vigorously stirred distilled water containing 1% (w/v) poly (vinyl alcohol). After 4 hours of stirring, the organic solvent evaporates from the polymer, and the resulting microspheres can be washed with water and dried overnight in a lyophilizer.

In embodiments where the spray-drying method is used, the polymer can be dissolved in methylene chloride. A known amount of drug is suspended (where the opioid antagonist is insoluble) or co-dissolved (where the opioid antagonist is soluble) in the polymer solution. The solution of the dispersion is then spray-dried. This method is used for small microspheres of between 1-10 microns.

In certain embodiments, a hot melt encapsulation method may be used. Using this method, the polymer may first be melted and then mixed with solid particles of drug that have been sieved to less than 50 microns. The mixture is suspended in a non-miscible solvent and, with continuous stirring, heated to 5°C above the melting point of the polymer. Once the emulsion is stabilized, it is cooled until the polymer particles solidify. The resulting microspheres are washed by decantation with petroleum ether to give a free-flowing powder. This technique is used for polyesters, polyanhydrides and polymers with high melting points and different molecular weights. The typical yield of microspheres in this process is about 80-90%. The resulting microspheres have a core-shell structure.

In order to create microspheres containing opioid antagonist, an organic or oil (discontinuous) phase and an aqueous phase may be combined. The organic and aqueous phases are largely or substantially immiscible, with the aqueous phase constituting the continuous phase of the emulsion. The organic phase includes the active agent and the wall forming polymer, i.e. the polymeric matrix material. The organic phase is prepared by dispersing the active opioid antagonist in the organic solvent(s). The organic and aqueous phases are preferably combined under the influence of a mixing means, preferably a static mixer.

Opioid antagonists useful in the present invention include, but are not limited to, nalorphine, nalorphine dinicotinate, naloxone, nalmephene, cyclazocine, levallorphan, naltrexone, nadide, cyclazocine, amiphenazole and pharmaceutically acceptable salts thereof and mixtures thereof. Preferably, the opioid antagonist is an orally bioavailable antagonist, e.g., naltrexone or pharmaceutically acceptable salt thereof. By utilizing a bioavailable antagonist, the transdermal device will deter both oral and parenteral abuse.

After the formation of the microspheres containing the opioid antagonist, the microspheres are incorporated into a transdermal delivery device containing an opioid agonist. Preferably, the microspheres are included in the transdermal delivery device so that they are substantially indistinguishable from the bulk of the opioid agonist-containing preparation (e.g., the microspheres can be imbedded in the matrix of the matrix delivery device). In certain embodiments, the opioid agonist is in a form that can be absorbed through human skin, i.e., the opioid agonist can be effectively administered via the transdermal route. In some embodiments, it may be necessary to further provide an absorption enhancer in order to facilitate transdermal absorption.

In the transdermal delivery devices of the present invention, the opioid agonist is available for absorption, passing through pores in the intact skin surface of typically less than 50 nm to provide sustained therapeutic levels over a prolonged period. Transdermal delivery devices that are prepared in accordance with the present invention may release the opioid agonist in accordance with first order pharmacokinetics (e.g., where the plasma concentrations of the opioid agonist increase over a specified time period), or in accordance with zero order pharmacokinetics (e.g., where plasma concentrations are maintained at relatively constant level over a specified time period), or with both first and zero order pharmacokinetics.

Opioid agonists that can be selected for use in the transdermal delivery devices of the present invention include any opioid agonists, mixed agonist-antagonists, or partial agonists, including but not limited to alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, remifentanil, sufentanil, tilidine, tramadol, pharmaceutically acceptable salts thereof, mixtures thereof, and the like.

In preferred embodiments, the opioid agonist is selected from the group consisting of transdermally administrable forms of fentanyl, buprenorphine, sufentanyl, hydrocodone, morphine, hydromorphone, oxycodone, codeine, levorphanol, meperidine, methadone, oxymorphone, dihydrocodeine, tramadol, pharmaceutically acceptable salts thereof, and mixtures thereof.

Any type of transdermal delivery device may be used in accordance with the methods of the present invention, as long as the desired pharmacokinetic and pharmacodynamic response(s) are attained over at least a 1 day period, e.g., from 2 to 8 days. Preferable transdermal delivery devices include, e.g., transdermal patches, transdermal plasters, transdermal discs, and the like.

In a preferred embodiment, the transdermal drug delivery device of the present invention is a patch, typically in the range of from about 1 to about 30 square centimeters, preferably 2 to 10 square centimeters. The term "patch" as used herein includes any product having a backing member and a pressure-sensitive adhesive face surface enabling adherence to the skin of a patient. Such products can be provided in various sizes and configurations, such as tapes, bandages, sheets, and the like.

In the transdermal delivery device of the present invention, the opioid agonist is preferably dispersed throughout a matrix (e.g., a polymer matrix). In such a matrix device, release of the opioid agonist can be predominantly controlled by diffusion of the opioid agonist out of the polymer, or by erosion of the polymer to release the opioid agonist, or by a combination of these two mechanisms. When the diffusion of opioid agonist is faster than the erosion of the polymer, drug release is controlled by diffusion. When polymer erosion is faster than diffusion of the opioid agonist, drug release is controlled by erosion of the polymer. If the delivery device is prepared with a surface-erosion polymer, the release of drug can be controlled by varying the amount of drug loaded into the device and/or by varying the geometric dimension of the delivery device.

Generally, the polymers used in the polymer matrix of the transdermal delivery device are those capable of forming thin walls or coatings through which the opioid agonist can pass at controlled rate. Examples of such polymers for use in preparing the polymer matrix include polyethylene, polypropylene, ethylene/propylene copolymers, ethylene/ethylacrylate copolymers, ethylenevinyl acetate copolymers, silicones, rubber, rubber-like synthetic homo-, co- or block polymers, polyacrylic esters and the copolymers thereof, polyurethanes, polyisobutylene, chlorinated polyethylene, polyvinylchloride, vinyl chloride-vinyl acetate copolymer, polymethacrylate polymer (hydrogen), polyvinylidene chloride, poly(ethylene terephthalate), ethylene-vinyl alcohol copolymer, ethylene-vinyloxyethanol copolymer, silicones including silicone copolymers such as polysiloxane-polymethacrylate copolymers, cellulose polymers (e.g., ethyl cellulose, and cellulose esters), polycarbonates, polytetrafluoroethylene and mixtures thereof.

Preferred materials for use in preparing the polymer matrix are silicone elastomers of the general polydimethylsiloxane structure, (e.g., silicone polymers). Preferred silicone polymers are those that cross-link and are pharmaceutically acceptable. For example, preferred materials for use in preparing the polymer matrix layer include silicone polymers that are cross-linkable copolymers having dimethyl and/or dimethylvinyl siloxane units that can be crosslinked using a suitable peroxide catalyst. Also preferred are those polymers consisting of block copolymers based on styrene and 1,3-dienes (particularly linear styrene-isoprene-block copolymers of styrene-butadiene-block copolymers), polyisobutylenes, polymers based on acrylate and/or methacrylate.

In certain embodiments, the polymer matrix includes a pharmaceutically acceptable cross-linking agent. Suitable crosslinking agents include, e.g., tetrapropoxysilane, among others.

Certain embodiments of the present invention include a polymer matrix layer comprising opioid agonist with intermixed microspheres of opioid antagonist. Preferably, for the opioid antagonist to become bioavailable, the integrity of the microspheres must be disrupted. The combination of microsphere with polymer matrix prevents release of the opioid antagonist from the microspheres embedded within the matrix in an intact device. Release of the opioid antagonist from microspheres may be further prevented by polymer coatings over the microspheres.

Preferably, the transdermal delivery device of the present invention comprises a backing layer made of a pharmaceutically acceptable material that is impermeable to the opioid agonist. The backing layer preferably serves as a protective cover for the opioid agonist and may also provide a support function. Examples of materials suitable for making the backing layer are films of high and low density polyethylene, polypropylene, polyvinylchloride, polyurethane, polyesters such as poly(ethylene phthalate), metal foils, metal foil laminates of such suitable polymer films, and textile fabrics. Preferably, the materials used for the backing layer are laminates of such polymer films with a metal foil such as aluminum foil. The backing layer can be any appropriate thickness that provides the desired protective and support functions. A suitable thickness will be, e.g., from about 10 to about 200 microns.

In certain alternative embodiments, the transdermal delivery device of the present invention can comprise microspheres are contained in a reservoir. In such a reservoir device, the opioid agonist and microspheres of opioid antagonist are dispersed in a reservoir (e.g., a liquid or gel reservoir), and a rate limiting biodegradable membrane is situated in the flow path of the drugs, thereby limiting the flux of the opioid agonist to the skin. Such a device can provide a constant release rate of opioid agonist, but serve to prevent release of the opioid antagonist. A transdermal delivery device utilizing a reservoir device can also have a backing layer, and optionally a removable protective layer as described above with the matrix device.

Preferred transdermal delivery devices used in accordance with the methods of the present invention preferably further include an adhesive layer to affix the delivery device to the skin of a patient for a desired period of administration, e.g., from 2 to 8 days. If the adhesive layer of the delivery device fails to provide adequate adhesion for the desired period of time, it is possible to maintain contact between the delivery device and the skin by, e.g., affixing the delivery device to the skin of the patient with adhesive tape, e.g., surgical tape. It is not critical for purposes of the present invention whether adhesion of the delivery device to the skin of the patient is achieved solely by the adhesive layer of the delivery device or by use of an external adhesive source, such as surgical tape, provided that the delivery device is adhered to the patient's skin for the requisite administration period. In all cases, however, the adhesive must allow for the patch to adhere firmly to the skin of the patient in need of treatment, but not be so strongly adhesive as to injure the patient when the patch is removed.

The adhesive layer can be selected from any adhesive known in the art that is pharmaceutically compatible with the delivery device. The adhesive is preferably hypoallergenic. Examples include a polyacrylic adhesive polymer, acrylate copolymer (e.g., polyacrylate) or polyisobutylene adhesive polymer. Other useful adhesives include silicones, polyisoalkylenes, rubbers, vinyl acetates, polybutadiene, styrene-butadiene (or isoprene)-styrene block copolymer rubber, acrylic rubber and natural rubber; vinyl-based high molecular weight materials such as polyvinyl alkyl ether, polyvinyl acetate; cellulose derivatives such as methylcellulose, carboxymethyl cellulose and hydroxypropyl cellulose; polysaccharides such as pullulan, dextrin and agar; and polyurethane elastomers and polyester elastomers. While many of these adhesives are virtually interchangeable, some combinations of a specific opioid analgesic and a specific adhesive may provide marginally better properties.

In some embodiments, the adhesive is a pressure-sensitive contact adhesive, which is preferably hypoallergenic.

In certain embodiments, the transdermal drug delivery material provides the functions of both drug-containing matrix and adhesive. In certain embodiments with a separate adhesive layer, the drug will be distributed throughout all the layers (with the exception of the backing layer) according to its relative affinity for the different environments offered by the different layers. The matrix "layer" may consist of more than a single sub-layer, with opioid loading in the different layers adjusted to optimize its delivery characteristics and opioid antagonist containing microspheres dispersed throughout. In such embodiments, the drug-containing matrix contacts the skin directly and the transdermal delivery device is held to the skin by a peripheral adhesive or the matrix itself.

In certain embodiments, the transdermal delivery device of the present invention optionally includes a permeation-enhancing agent. Permeation-enhancing agents are compounds that promote penetration and/or absorption of the opioid agonist through the skin into the blood stream of the patient. As a result of these penetration enhancers, almost any drug, to some degree, can be administered transdermally. Permeation-enhancing agents are generally characterized to be from the group of monovalent branched or unbranched aliphatic, cycloaliphatic or aromatic alcohols of 4-12 carbon atoms; cycloaliphatic or aromatic aldehydes or ketones of 4-10 carbon atoms, cycloalkanoyl amides of C₁₀₋₂₀ carbons, aliphatic, cycloaliphatic and aromatic esters, N,N-di-lower alkylsulfoxides, unsaturated oils, terpenes and glycol silicates. A non-limiting list of permeation-enhancing agents includes polyethylene glycols, surfactants, and the like.

Permeation of the opioid agonist can be also be enhanced by occlusion of the delivery device after application to the desired site on the patient with, e.g. an occlusive bandage. Permeation can also be enhanced by removing hair from the application site by, e.g. clipping, shaving or use of a depilatory agent. Another approach to enhancing permeation is by the application of heat to the site of the adhered patch, such as with an infrared lamp. Other approaches to enhancing the permeation of opioid agonist includes the use of iontophoretic means.

In certain embodiments, the transdermal delivery device includes a softening agent to modify the skin at the point of adhesion to promote drug absorption. Suitable softening agents include higher alcohols such as dodecanol, undecanol, octanol, esters of carboxylic acids, wherein the alcohol component may also be a polyethoxylated alcohol, diesters of dicarboxylic acids, such as di-n-butyladiapate, and triglycerides particularly medium-chain triglycerides of the caprylic/capric acids or coconut oil. Further examples of suitable softening agents are multivalent alcohols, e.g., levulinic acid, caprylic acids, glycerol and 1,2-propanediol, which can also be etherified by polyethylene glycols.

In certain embodiments, a solvent for the opioid agonist is included in the transdermal delivery device of the present invention. Preferably, the solvent dissolves the opioid agonist to a sufficient extent, thereby avoiding complete salt formation. A non-limiting list of suitable solvents includes those with at least one acidic group. Monoesters of dicarboxylic acids such as monomethylglutarate and monomethyladipate are particularly suitable.

Other pharmaceutically acceptable compounds that may be included in the transdermal delivery device of the present invention include viscosity enhancing agents, such as cellulose derivatives, natural or synthetic gums, such as guar gum, and the like.

In certain embodiments of the present invention, the transdermal delivery device further includes a removable protective layer. The removable protective layer is removed prior to application, and can consist of materials used for the production of the backing layer described above, provided that they are rendered removable, e.g., by a silicone treatment. Other examples of removable protective layers are polytetra-fluoroethylene, treated paper, allophane, polyvinyl chloride, and the like. Generally, the removable protective layer is in contact with the adhesive layer, and provides a convenient means of maintaining the integrity of the adhesive layer until the desired time of application.

It is well understood in the art of transdermal delivery devices that in order to maintain a desired flux rate for a desired dosing period, it is necessary to include an "overage" of active agent in the transdermal delivery device in an amount that is substantially greater than the amount to be delivered to the patient over the desired time period. For example, to maintain the desired flux rate for a three day time period, it is considered necessary to include in a transdermal delivery device much greater than what would otherwise be 100% of a three-day dose of the active agent. The remainder of the active agent remains in the transdermal delivery device. Only that portion of active agent that exits the transdermal delivery device becomes available for absorption into the skin.

The term "overage" means for the purposes of the present invention the amount of opioid analgesic contained in a transdermal delivery device that is not delivered to the patient. The overage is necessary for creating a sufficient concentration gradient by which the active agent will migrate from the transdermal delivery device through a patient's skin to produce a sufficient therapeutic effect.

Preferably, the transdermal delivery device of the present invention is used for prolonged dosing, releasing the opioid agonist in a constant or pulsed manner to the patient while the opioid antagonist contained in the microspheres remains unreleasable or substantially unreleasable.

Non-opioid analgesics that may be included in combination with the opioid agonist are, e.g., acetaminophen, phenacetin and non-steroidal anti-inflammatory agents. Suitable non-steroidal anti-inflammatory agents include aspirin, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid tolfenamic acid, diflurisal, flufenisal, piroxicam, sudoxicam or isoxicam, pharmaceutically acceptable salts thereof, and mixtures thereof. Other suitable non-steroidal anti-inflammatory agents include cox-2 inhibitors such as celecoxib, DUP-697, flosulide, meloxicam, 6-MNA, L-745337, rofecoxib, nabumetone, nimesulide, NS-398, SC-5766, T-614, L-768277, GR-253035, JTE-522, RS-57067-000, SC-58125, SC-078, PD-138387, NS-398, flosulide, D-1367, SC-5766, PD-164387, etoricoxib, valdecoxib, parecoxib, pharmaceutically acceptable salts thereof, and mixtures thereof

Other active agents that may be combined with the opioid agonist can be, e.g, antiemetic/antivertigo agents such as chlorpromazine, perphenazine, triflupromazine, prochlorperazine, triethylperazine, metoclopropramide, cyclizine, meclizine, scopolamine, diphenhydramine, buclizine, dimenhydrate, and trimethobenzamide; 5-HT₃ receptor antagonists such as ondansetron, granisetron, and dolasetron; anti-anxiety agents such as meprobamate, benzodiazepines, buspirone, hydroxyzine, and doxepin, and the like.

It is contemplated that previously known transdermal delivery devices can be modified by including in the matrix, reservoir, and/or adhesive layers opioid-antagonist-containing microspheres as described above, so as to decrease the potential for abuse of such devices. For example, the transdermal delivery devices for use in accordance with the present invention can use certain aspects described in U.S. Patent No. 5,240,711 to Hille, et. al.; U.S. Patent No. 5,225,199 to Hidaka et al.; U.S. Patent No. 4,588,580 to Gale et. al.; U.S. Patent No. 5,069,909 to Sharma et al.; U.S. Patent No. 4,806,341 to Chien et al.; U.S. Patent No. 5,026,556 to Drust et al.; and McQuinn, R. L. et al, "Sustained Oral Mucosal Delivery in Human Volunteers" J. Controlled Release; (34) 1995 (243-250).

The following examples are not meant to limit the invention in any manner.

### EXAMPLE 1

Using the procedure disclosed in this example, multiple batches of naltrexone-loaded microspheres were prepared using different molecular weight Lactide/Glycolide (65:35) polymers (40 KD, 40 KD with 0.01% calcium chloride, 50:50 blend of 40 KD and low molecular weight (about 10 KD) and 11 KD)

Naltrexone-loaded microspheres were fabricated using a water-in-oil-in-water (w/o/w) double-emulsion solvent extraction / evaporation technique. In this process, naltrexone was dissolved in phosphate-buffered saline (PBS) (pH 7.4) solution containing 0.05% (w/ v) polyvinylalcohol (PVA) as an emulsifier and mixed with ethyl acetate containing poly(lactic-co-glycolic acid) (PLGA). The emulsification was carried out by sonication for 15 seconds. The resulting emulsion was further injected into PBS (pH 7.4) containing 0.05% (w/ v) PVA as an emulsifier to produce a double w/o/w emulsion. The dispersion was then stirred at a constant temperature for 30 minutes. In order to extract ethyl acetate from the first emulsion into the external phase, a second buffer solution (pH 7.4) containing 0.05% (w/ v) PVA was added continuously at a rate of 3 ml / minute. The temperature of the second emulsion throughout the solvent extraction / evaporation stage was maintained constant using a low-temperature circulator. The resulting naltrexone-loaded microspheres were collected by vacuum-filtration and washed three times with PBS. The microspheres were then vacuum-dried overnight and stored at 4C.

The load of naltrexone for the microspheres is set forth below in Table 1.

**TABLE 1**

| Polymer | Load of naltrexone of entire microsphere |
|---|---|
| 40 KD | 42.2% |
| 40 KD and 0.01 % Calcium chloride | 42.3% |
| 50:50 blend of 40 KD and low molecular weight (about 10 KD) | 39.3% |
| 11 KD | 28.8% |

### EXAMPLE 2

The microsphere prepared in Example 1 were exposed to simulated extraction conditions to determine the degree of in-vitro release of naltrexone from the microspheres. The extractions were performed using 0.5N NaCl, pH 6.5 phosphate buffer. The sample size was 100 mg microspheres and the naltrexone release was measures at 0.5, 1 and 4 hours. The results are set forth in Table 2 and Figure 5.

**TABLE 2**

| Polymer | Ntx Content (per 100 mg microsphere) | Release at 30 minutes | Release at 1 hour | Release at 4 hours |
|---|---|---|---|---|
| 40 KD | 28.8 mg as Base | 54.7% | 57.8% | 64.6% |
| 40 KD and 0.01 % Calcium chloride | 42.2 mg as Base | 0 | 1.2% | 1.2% |
| 50:50 blend of 40 KD and low molecular weight (about 10 KD) | 39.3 mg as Base | 6.4% | 7.6% | 14.0% |
| 11 KD | 42.3 mg as Base | 2.4% | 3.5% | 5.9% |

Based on the amount of antagonist released from any given microsphere formulation, the amount of antagonist loaded into the microspheres can be adjusted in order to obtain the release of a desired amount upon tampering.

### EXAMPLE 3 (Prophetic)

Microspheres are prepared as follows. Naltrexone is mixed with requisite amounts of gelatin, Tween 80 and water, and heated. The mixture is then dispersed in a mixture of aluminum monostearate, Span 80 and soybean oil to form a microemulsion. The microemulsion is homogenized by a microfluidizer. Thereafter, the microemulsion is dispersed in a PLGA-acetonitrile solution. The acetonitrile is then removed from the emulsion by evaporation under atmospheric pressure, thereby forming microspheres containing naltrexone to be incorporated into a transdermal delivery device.

### EXAMPLE 4 (Prophetic)

A transdermal patch is prepared in accordance with the disclosure of WO 96/19975 to LTS GMBH, published July 4, 1996, with the addition of naltrexone-containing microspheres prepared in accordance with Example 1, as follows:

The following are homogenized: 1.139 g of a 47.83 w/w % polyacrylate solution with a self cross-linking acrylate copolymer containing 2-ethylhexylacrylate, vinyl acetate, acrylic acid (dissolving agent:ethylacetate:heptan:isopropanol:toluol:acetylacetonate in the ratio of 37:26:26:4:1), 100 g laevulinic acid, 150 g oleyloleate, 100 g polyvinylpyrrolidone, 150 g ethanol, 200 g ethyl acetate and 100 g buprenorphine base. The mixture is stirred for about 2 hours and then examined visually to confirm that all solid substances have been dissolved. Evaporation loss is controlled by method of weighing back and making up for the solvent with addition of of ethylacetate, if necessary. Thereafter, the mixture is combined with the naltrexone microspheres prepared as described above in Example 1. This mixture is then transferred to a 420 mm wide transparent polyester foil. The solvent is removed by drying with heated air. Thereafter, the sealing film is covered with a polyester foil. A surface of about 16 cm² is cut with the help of the appropriate cutting tool.

## Claims

1. A transdermal delivery device comprising:
a drug containing layer comprising an effective amount of an opioid agonist and a plurality of microspheres dispersed in the drug containing layer, the microspheres comprising an opioid antagonist and wherein the microspheres are in a mean size of from 1 to 300 µm in diameter.

2. The transdermal delivery device of claim 1, wherein the plurality of microspheres comprise the opioid antagonist dispersed in a polymeric matrix.

3. The transdermal delivery device of claim 1, wherein the microspheres further comprise a polymer selected from the group consisting of polyesters, polyethers, poly(orthoesters), polysaccharides, cyclodextrins, chitosans, poly (Σ-caprolactones), polyanhydrides, albumin, blends and copolymers thereof and mixtures thereof.

4. The transdermal delivery device of claim 1, wherein the microspheres consist essentially of the opioid antagonist and a polymer selected from the group consisting of polyesters, polyethers, poly(orthoesters), polysaccharides, cyclodextrins, chitosans, poly (E-caprolactones), polyanhydrides, albumin, blends and copolymers thereof.

5. The transdermal delivery device of claim 1, wherein the microspheres consist of the opioid antagonist dispersed in a polymeric matrix.

6. The transdermal delivery device of claim 1, wherein the microspheres are in a mean size of from 125 to 200 microns, from 50 to 100 microns, from 1 to 200 microns, or from 1 to 100 microns in diameter.

7. The transdermal delivery device of claim 1, wherein the opioid antagonist becomes releasable if the transdermal delivery device is chewed, soaked, punctured, torn, or subjected to any other treatment which disrupts the integrity of the microspheres.

8. The transdermal delivery device of claim 1, wherein the effect of the opioid agonist is at least partially blocked when the delivery device is chewed, crushed or dissolved in a solvent, or subject to any other treatment which disrupts the integrity of the microspheres, and administered orally, intranasally, parenterally or sublingually.

9. The transdermal delivery device of claim 1, wherein the opioid antagonist is in the form of stable crystalline particles.

10. The transdermal delivery device of claim 1, wherein the microspheres are uniformly dispersed in within the drug layer.

11. The transdermal delivery device of claim 1, wherein the opioid agonist is selected from the group consisting of fentanyl, sufentanyl, buprenorphine, hydrocodone, morphine, hydromorphone, oxycodone, codeine, levorphanol, meperidine, methadone, oxymorphone, dihydrocodeine, tramadol, pharmaceutically acceptable salts thereof, and mixtures thereof.

12. The transdermal delivery device of claims 1 or 11 wherein the opioid antagonist is selected from the group consisting of naltrexone, naloxone, nalmephene, diprenorphine, nalmexone, cyprenorphine, alazocine, oxilorphan, cyclophan, nalorphine, nalbuphine, buprenorphine butorphanol, cyclazocine, pentazocine, levallorphan, pharmaceutically acceptable salts thereof, and mixtures thereof; and preferably is naltrexone or a pharmaceutically acceptable addition salt thereof.

13. The transdermal delivery device of claim 1, wherein the opioid analgesic is in an effective amount to provide analgesia for a period of time of from 2 to 8 days when affixed to the skin of the human patient.

14. The transdermal delivery device of claim 1, wherein the drug containing layer is a matrix layer, and the matrix preferably comprises a material selected from the group consisting of polyethylene, polypropylene, ethylene/propylene copolymers, ethylene/ethylacrylate copolymers, ethylenevinyl acetate copolymers, silicones, rubber, rubber-like synthetic homo-, co- or block polymers, polyacrylic esters and the copolymers thereof, polyurethanes, polyisobutylene, chlorinated polyethylene, polyvinylchloride, vinyl chloride-vinyl acetate copolymer, polymethacrylate polymer (hydrogel), polyvinylidene chloride, poly(ethylene terephthalate), ethylene-vinyl alcohol copolymer, ethylene-vinyloxyethanol copolymer, silicones (e.g., silicone copolymers such as polysiloxane-polymethacrylate copolymers), cellulose polymers (e.g., ethyl cellulose, and cellulose esters), polycarbonates, polytetrafluoroethylene and mixtures thereof.

15. The transdermal delivery device of claim 2, where the matrix is selected from the group consisting of silicone polymers, silicone polymers that are cross-linkable, copolymers having dimethyl and/or dimethylvinyl siloxane units which can be crosslinked, block copolymers based on styrene and 1,3-dienes, polyisobutylenes, polymers based on acrylate and/or methacrylate.

16. The transdermal delivery device of claim 14, further comprising an adhesive layer adjacent to, and in contact with, the matrix layer and permeable to the therapeutically active agent.

17. The transdermal delivery device of claim 1, wherein the drug containing layer is an adhesive layer and/or a reservoir layer.

18. The transdermal delivery device of claim 17, wherein the reservoir layer further comprising a rate controlling membrane layer superimposed on the reservoir layer and substantially coextensive therewith; and preferably further comprising an adhesive layer adjacent to, and in contact with, the membrane layer and permeable to the therapeutically active agent.

19. The transdermal delivery device of claim 16, further comprising a protective layer covering and adhearing to the adhesive layer and removable therefrom for the use of the transdermal delivery device.

20. The transdermal delivery device of claim 1, wherein the transdermal delivery device is a device selected from the group consisting of a transdermal patch, a transdermal plaster, a transdermal disc, and iontophoretic transdermal device.

21. A use of a transdermal delivery device in the manufacture of a medicament for the treatment of pain comprising applying the transdermal delivery device according to claim 1 to the skin of a patient.

22. The use of a transdermal delivery device in the manufacture of a medicament for preventing abuse of an opioid agonist transdermal delivery device comprising making a transdermal delivery device according to claim 1.

23. A use of a transdermal delivery device according to any one of claims 1 to 20 in the preparation of a medicament to provide analgesia to a patient in need thereof.

## Patentansprüche

1. Transdermale Verabreichungsvorrichtung, umfassend:
eine Wirkstoff enthaltende Schicht, die eine wirksame Menge eines Opioid-Agonisten umfasst, und eine Vielzahl von Mikrokügelchen, die in der Wirkstoff enthaltenden Schicht dispergiert sind, wobei die Mikrokügelchen einen Opioid-Antagonisten enthalten und wobei die Mikrokügelchen in einer mittleren Größe von 1 bis 300 µm im Durchmesser vorliegen.

2. Transdermale Verabreichungsvorrichtung gemäß Anspruch 1, wobei die Vielzahl der Mikrokügelchen den Opioid-Antagonisten in einer Polymermatrix dispergiert umfassen.

3. Transdermale Verabreichungsvorrichtung gemäß Anspruch 1, wobei die Mikrokügelchen weiterhin ein Polymer umfassen, ausgewählt aus der Gruppe bestehend aus Polyester, Polyether, Poly(orthoester), Polysaccharide, Cyclodextrine, Citosane, Poly(ε-caprolactone), Polyanhydride, Albumin, Blends und Copolymere davon und Gemische davon.

4. Transdermale Verabreichungsvorrichtung gemäß Anspruch 1, wobei die Mikrokügelchen im wesentlichen aus dem Opioid-Antagonisten und einem Polymer, ausgewählt aus der Gruppe bestehend aus Polyester, Polyether, Poly(orthoester), Polysaccharide, Cyclodextrine, Citrosane, Poly(ε-caprolactone), Polyanhydride, Albumin, Blends und Copolymeren davon, bestehen.

5. Transdermale Verabreichungsvorrichtung gemäß Anspruch 1, wobei die Mikrokügelchen aus dem in einer Polymermatrix dispergierten Opioid-Antagonisten bestehen.

6. Transdermale Verabreichungsvorrichtung gemäß Anspruch 1, wobei die Mikrokügelchen in einer mittleren Größe von 125 bis 200 µm, von 50 bis 100 µm, von 1 bis 200 µm oder von 1 bis 100 µm im Durchmesser vorliegen.

7. Transdermale Verabreichungsvorrichtung gemäß Anspruch 1, wobei der Opioid-Antagonist freisetztbar wird, wenn die transdermale Verabreichungsvorrichtung gekaut, getränkt, durchlöchert, zerrissen oder irgendeiner anderen Behandlung unterzogen wird, die die Integrität der Mikrokügelchen beeinträchtigt.

8. Transdermale Verabreichungsvorrichtung gemäß Anspruch 1, wobei die Wirkung des Opioid-Agonisten zumindest teilweise blockiert wird, wenn die Verabreichungsvorrichtung gekaut, zerstoßen oder in einem Lösungsmittel gelöst wird oder irgendeiner anderen Behandlung unterzogen wird, die die Integrität der Mikrokügelchen beeinträchtigt, und oral, intranasal, parenteral oder sublingual verabreicht wird.

9. Transdermale Verabreichungsvorrichtung gemäß Anspruch 1, wobei der Opioid-Antagonist in Form von stabilen kristallinen Teilchen vorliegt.

10. Transdermale Verabreichungsvorrichtung gemäß Anspruch 1, wobei die Mikrokügelchen einheitlich innerhalb der Wirkstoffschicht dispergiert sind.

11. Transdermale Verabreichungsvorrichtung gemäß Anspruch 1, wobei der Opioid-Agonist ausgewählt ist aus der Gruppe bestehend aus Fentanyl, Sufentanyl, Buprenorphin, Hydrocodon, Morphin, Hydromorphon, Oxycodon, Codein, Levorphanol, Meperidin, Methadon, Oxymorphon, Dihydrocodein, Tramadol, pharmazeutisch verträglichen Salzen davon und Gemischen davon.

12. Transdermale Verabreichungsvorrichtung gemäß Anspruch 1, oder 11, wobei der Opioid-Antagonist ausgewählt ist aus der Gruppe bestehend aus Naltrexon, Naloxon, Nalmephen, Diprenorphin, Nalmexon, Cyprenorphin, Alazocin, Oxylorphan, Cyclophan, Nalorphin, Nalbuphin, Buprenorphin-Butorphanol, Cyclazocin, Pentazocin, Levallorphan, pharmazeutisch verträglichen Salzen davon und Gemischen davon; und vorzugsweise Naltrexon oder ein pharmazeutisch verträgliches Additionssalz davon ist.

13. Transdermale Verabreichungsvorrichtung gemäß Anspruch 1, wobei das Opioid-Schmerzmittel in einer wirksamen Menge vorliegt, die Analgesie für eine Zeitdauer von 2 bis 8 Tage bereitstellt, wenn sie auf der Haut eines menschlichen Patienten angebracht wird.

14. Transdermale Verabreichungsvorrichtung gemäß Anspruch 1, wobei die wirkstoffenthaltende Schicht eine Matrixschicht ist, und die Matrix vorzugsweise ein Material umfasst, ausgewählt aus der Gruppe bestehend aus Polyethylen, Polypropylen, Ethylen/Propylencopolymeren, Ethylen/Ethylacrylat-Copolymeren, Ethylenvinylacetat-Copolymeren, Silikonen, Gummi, gummiähnlichen synthetischen Homo-, Co- oder Blockpolymeren, Polyacrylestern und Copolymere davon, Polyurethanen, Polyisobutylen, Chlorinat-Polyethylen, Polyvinylchlorid, Vinyl-Chlorid-Vinylacetat-Copolymeren, Polymethacrylat-Polymer (Hydrogel), Polyvinylidenchloird, Poly(ethylenterephthalat), Ethylenvinylalkohol-Copolymeren, Ethylenvinyloxyethanol-Copolymern, Silikon (z.B. Silicon-Copolymere wie Polysiloxan-Polymethacrylat-Copolymere), Zellulosepolymere (z.B. Ethylcellulose und Celluloseester), Polycarbonat, Polytetrafluroethylen und Gemischen davon.

15. Transdermale Verabreichungsvorrichtung gemäß Anspruch 2, wobei die Matrix ausgewählt ist aus der Gruppe bestehend aus Silikonpolymeren, Silikonpolymeren, die vernetzbar sind, Copolymeren mit Dimethyl- und/oder Dimethylvinylsiloxaneinheiten, die vernetzt werden können, Blockcopolymeren, basierend auf Styrol und 1,3-Dienen, Polyisobutylene, Polymeren, basierend auf Acrylat und/oder Methacrylat.

16. Transdermale Verabreichungsvorrichtung gemäß Anspruch 14, weiterhin eine der Matrixschicht benachbarte und in Kontakt mit dieser stehende Klebstoffschicht umfassend, welche für den therapeutischen Wirkstoff durchlässig ist.

17. Transdermale Verabreichungsvorrichtung gemäß Anspruch 1, wobei die wirkstoffenthaltende Schicht eine Klebstoffschicht und/oder eine Reservoirschicht ist.

18. Transdermale Verabreichungsvorrichtung gemäß Anspruch 17, wobei die Reservoirschicht weiterhin eine geschwindigkeitskontrollierende Membranschicht umfasst, welche auf der Reservoirschicht aufgebracht ist und eine im wesentlichen gleiche Ausdehnung aufweist; und vorzugsweise weiterhin eine der Membranschicht benachbarte und in Kontakt mit dieser stehende Klebstoffschicht umfassend, welche für den therapeutischen Wirkstoff durchlässig ist.

19. Transdermale Verabreichungsvorrichtung gemäß Anspruch 16, weiterhin eine Schutzschicht umfassend, welche die Klebstoffschicht bedeckt und dieser anhaftet und davon für die Verwendung der transdermalen Verabreichungsvorrichtung entfernt werden kann.

20. Transdermale Verabreichungsvorrichtung gemäß Anspruch 1, wobei die transdermale Verabreichungsvorrichtung eine Vorrichtung ist, ausgewählt aus der Gruppe bestehend aus einem transdermalen Pflaster, einem transdermalen Gips, einer transdermalen Scheibe und einer iontophoretischen transdermalen Vorrichtung.

21. Verwendung einer transdermalen Verabreichungsvorrichtung bei der Herstellung eines Medikaments zur Behandlung von Schmerz, umfassend Aufbringen der transdermalen Verabreichungsvorrichtung gemäß Anspruch 1 auf die Haut eines Patienten.

22. Verwendung einer transdermalen Verabreichungsvorrichtung bei der Herstellung eines Medikaments zur Verhinderung des Missbrauchs einer transdermalen Verabreichungsvorrichtung mit Opioid-Agonists, umfassend Herstellen einer transdermalen Verabreichungsvorrichtung gemäß Anspruch 1.

23. Verwendung einer transdermalen Verabreichungsvorrichtung gemäß einem der Ansprüche 1 bis 20 bei der Herstellung eines Medikaments zum Bereitstellen von Analgesie bei einem Patienten, der dessen Bedarf.

## Revendications

1. Dispositif d'administration transdermique comprenant :
une couche contenant un médicament comprenant une quantité efficace d'un agoniste opioïde et une pluralité de microsphères dispersées dans la couche contenant le médicament, les microsphères contenant un antagoniste opioïde et dans lequel les microsphères présentent une taille moyenne de 1 à 300 µm de diamètre.

2. Dispositif d'administration transdermique de la revendication 1, dans lequel la pluralité de microsphères contiennent l'antagoniste opioïde dispersé dans une matrice polymère.

3. Dispositif d'administration transdermique de la revendication 1, dans lequel les microsphères comprennent en outre un polymère sélectionné dans le groupe constitué de polyesters, de polyéthers, de poly(orthoesters), de polysaccharides, de cyclodextrines, de chitosans, de poly(Σ-caprolactones), de polyanhydrides, d'albumine, de mélanges et de copolymères de ceux-ci et de mélanges de ceux-ci.

4. Dispositif d'administration transdermique de la revendication 1, dans lequel les microsphères sont essentiellement constituées de l'antagoniste opioïde et d'un polymère sélectionné dans le groupe constitué de polyesters, de polyéthers, de poly(orthoesters), de polysaccharides, de cyclodextrines, de chitosans, de poly(Σ-caprolactones), de polyanhydrides, d'albumine, de mélanges et de copolymères de ceux-ci.

5. Dispositif d'administration transdermique de la revendication 1, dans lequel les microsphères sont constituées de l'antagoniste opioïde dispersé dans une matrice polymère.

6. Dispositif d'administration transdermique de la revendication 1, dans lequel les microsphères présentent une taille moyenne de 125 à 200 microns, de 50 à 100 microns, de 1 à 200 microns ou de 1 à 100 microns de diamètre.

7. Dispositif d'administration transdermique de la revendication 1, dans lequel l'antagoniste opioïde devient libérable si le dispositif d'administration transdermique est mastiqué, imprégné, percé, déchiré ou soumis à tout autre traitement qui rompt l'intégrité des microsphères.

8. Dispositif d'administration transdermique de la revendication 1, dans lequel l'effet de l'agoniste opioïde est au moins partiellement bloqué lorsque le dispositif d'administration est mastiqué, écrasé ou dissous dans un solvant, ou bien soumis à tout autre traitement qui rompt l'intégrité des microsphères et administré par voie orale, intranasale, parentérale ou sublinguale.

9. Dispositif d'administration transdermique de la revendication 1, dans lequel l'antagoniste opioïde se présente sous la forme de particules cristallines stables.

10. Dispositif d'administration transdermique de la revendication 1, dans lequel les microsphères sont uniformément dispersées dans la couche de médicament.

11. Dispositif d'administration transdermique de la revendication 1, dans lequel l'agoniste opioïde est sélectionné dans le groupe constitué du fentanyle, du sufentanyle, de la buprénorphine, de l'hydrocodone, de la morphine, de l'hydromorphone, de l'oxycodone, de la codéine, du lévorphanol, de la mépéridine, de la méthadone, de l'oxymorphone, de la dihydrocodéine, du tramadol, de sels pharmaceutiquement acceptables de ceux-ci et de mélanges de ceux-ci.

12. Dispositif d'administration transdermique des revendications 1 ou 11, dans lequel l'antagoniste opioïde est sélectionné dans le groupe constitué de la naltrexone, de la naloxone, du nalméphène, de la diprénorphine, de la nalmexone, de la cyprénorphine, de l'alazocine, de l'oxilorphan, du cyclophan, de la nalorphine, de la nalbuphine, de la buprénorphine, du butorphanol, de la cyclazocine, de la pentazocine, du levallorphan, de sels pharmaceutiquement acceptables de ceux-ci et de mélanges de ceux-ci ; et de préférence la naltrexone ou un sel d'addition pharmaceutiquement acceptable de celle-ci.

13. Dispositif d'administration transdermique de la revendication 1, dans lequel l'analgésique opioïde se trouve dans une quantité efficace afin de provoquer une analgésie pendant une période de 2 à 8 jours lorsqu'il est fixé sur la peau du patient humain.

14. Dispositif d'administration transdermique de la revendication 1, dans lequel la couche contenant le médicament est une couche matricielle et la matrice comprend de préférence un matériau sélectionné dans le groupe constitué du polyéthylène, du polypropylène, de copolymères éthylène/propylène, de copolymères éthylène/éthylacrylate, de copolymères éthylène/acétate de vinyle, de silicones, de caoutchoucs, d'homopolymères, de copolymères ou de polymères séquencés synthétiques de type caoutchouc, d'esters polyacryliques et de copolymères de ceux-ci, de polyuréthanes, du polyisobutylène, du polyéthylène chloré, du polychlorure de vinyle, du copolymère chlorure de vinyle / acétate de vinyle, d'un polymère polyméthacrylate (hydrogel), du chlorure de polyvinylidène, du poly (éthylène téréphtalate), d'un copolymère éthylène / alcool vinylique, d'un copolymère éthylène / vinyloxyéthanol, de silicones (par exemple des copolymères de silicone tels que des copolymères polysiloxane / polyméthacrylate), des polymères de cellulose (par exemple l'éthylcellulose et les esters de cellulose), de polycarbonates, du polytétrafluoréthylène et de mélanges de ceux-ci.

15. Dispositif d'administration transdermique de la revendication 2, dans lequel la matrice est sélectionnée dans le groupe constitué de polymères de silicone, de polymères de silicone réticulables, de copolymères ayant des unités diméthyl et/ou diméthylvinyl siloxane réticulables, de copolymères séquencés basés sur le styrène et de 1,3-diènes, des polyisobutylènes, de polymères basés sur l'acrylate et/ou le méthacrylate.

16. Dispositif d'administration transdermique de la revendication 14, comprenant en outre une couche adhésive adjacente et en contact avec la couche matricielle et perméable à l'agent thérapeutiquement actif.

17. Dispositif d'administration transdermique de la revendication 1, dans lequel la couche contenant le médicament est une couche adhésive et/ou une couche réservoir.

18. Dispositif d'administration transdermique de la revendication 17, dans lequel la couche réservoir comprend en outre une couche à membrane contrôlant le taux superposée à la couche réservoir et sensiblement coextensive par rapport à celle-ci ; et comprend en outre, de préférence, une couche adhésive adjacente et en contact avec la couche à membrane et perméable à l'agent thérapeutiquement actif.

19. Dispositif d'administration transdermique de la revendication 16, comprenant en outre une couche protectrice recouvrant et adhérant à la couche adhésive et amovible par rapport à celle-ci pour l'utilisation du dispositif d'administration transdermique.

20. Dispositif d'administration transdermique de la revendication 1, dans lequel le dispositif d'administration transdermique est un dispositif sélectionné dans le groupe constitué d'un patch transdermique, d'un sparadrap transdermique, d'un disque transdermique et d'un dispositif transdermique iontophorétique.

21. Utilisation d'un dispositif d'administration transdermique pour la fabrication d'un médicament pour le traitement de la douleur comprenant l'application du dispositif d'administration transdermique selon la revendication 1 sur la peau d'un patient.

22. Utilisation d'un dispositif d'administration transdermique pour la fabrication d'un médicament permettant de prévenir l'abus d'un dispositif d'administration transdermique d'un agoniste opioïde comprenant la réalisation d'un dispositif d'administration transdermique selon la revendication 1.

23. Utilisation d'un dispositif d'administration transdermique selon l'une des revendications 1 à 20 pour la préparation d'un médicament provoquant une analgésie chez un patient qui en a besoin.
